# EUROPEAN PATENT APPLICATION

(11) **EP 1 921 449 A1**
(43) Date of publication of application: **14.05.2008**
(21) Application number: 06023094.3
(22) Date of filing: 07.11.2006
(51) Int. Cl.: G01N 33/24, A01G 25/16

(54) **Device for determining soil moisture content**

(71) Applicant: Sensirion AG, 8712 Stäfa (CH)
(72) Inventor: Rothacher, Urs, 8006 Zürich (CH); Mayer, Felix, 8712 Stäfa (CH)
(74) Representative: Blum, Rudolf Emil

(57) **Abstract**

The device comprises a housing (2) with two humidity sensors (11, 12). A first opening (3) of the housing (2) is placed onto or into the soil (6), while a second opening (4) is located outside the soil in communication with the environment. A chamber (5) extends in the housing (2) between the first and the second opening. This chamber (5) allows humidity to diffuse between the openings (3, 4). The humidity sensors (11, 12) are located to measure the humidity at two spaced apart locations within the chamber (5). The difference of humidity measured at these two locations is a measure of soil moisture content.

## Description

The invention relates to a device for determining soil moisture content.

It has been known to measure soil moisture content by means of suitable devices. Such devices can e.g. issue an alert if the soil is too dry, which in turn may, for example, be used to initiate automatic or manual irrigation.

Conventional devices of this type e.g. use a humidity sensor embedded in the soil. This sensor is able to detect the humidity within the soil. It has been found, however, that such sensors tend to be saturated if the humidity is high while they measure, at best, only very low humidity once the soil has started to dry. Also, the soil moisture content that they report is strongly dependent on the actual position of the sensor within the soil.

It is an object of the present invention to provide a humidity sensor that generates a signal that is more robust and reliable.

This object is achieved by the device of the independent claim.

Accordingly, the device comprises a housing with at least one humidity sensor. A first opening of the housing is placed onto or into the soil, while a second opening is located outside the soil in communication with the environment. A chamber is formed in the housing between the first and the second opening. This chamber allows humidity to diffuse between the openings. The humidity sensor is located to measure the humidity at a location in the chamber.

It has been found that this set-up provides a more reliable signal. The chamber allows humidity to diffuse from the first to the second opening, which leads to a humidity gradient between the first and second opening.

By comparing the humidity at the location of the sensor to a reference humidity, which can e.g. be a fixed humidity or a humidity measured by a second sensor, a more accurate result can be obtained. Advantageously, the device comprises a first and a second humidity sensor and compares the humidities measured by the same.

The second sensor can be located to measure the humidity at a second location in the chamber, which second location is at a distance from the first location. Hence, if the presence of water in the soil leads to a diffusion and therefore a gradient of humidity in the chamber, a comparison of the humidities measured by the two sensors allows to obtain a very reliable result.

The second sensor can also be located outside the chamber for measuring an ambient humidity, which also allows to obtain a very reliable result by comparing the humidities measured by the two sensors.

The invention also relates to the use of this type of device for determining soil moisture content.

The term "soil moisture content" is used herein in a broad manner. It can be understood to designate a numeric value indicating at least an approximation of the water content of the soil per volume, or it can also be understood to designate a qualitative measure, such as a false/true-valued quantity indicating if the soil needs irrigating.

Other advantageous embodiments of the invention are disclosed in the dependent claims as well as in the following description and the enclosed figures:
Fig. 1 shows a sectional view of a first embodiment probe for determining soil moisture content and a block diagram of the circuitry thereof and
Fig. 2 is a second embodiment of such a probe.

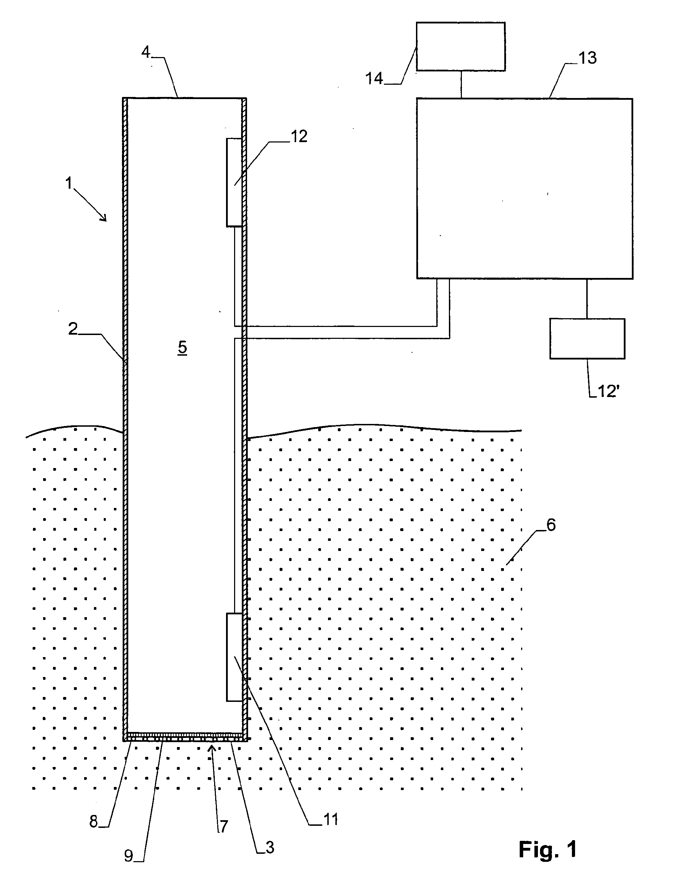

The device shown in Fig. 1 comprises a probe 1 having a tubular housing 2. Tubular housing 2 can e.g. have round or polygonal cross section. It may have constant or varying cross section, and in may in particular also be conical. It has a first opening 3 at its bottom end and a second opening 4 at its top end and encloses an e.g. cylindrical, elongate chamber 5 extending between both openings.

First opening 3, which is to be embedded into or placed onto the surface of a section of soil 6, can be covered by a permeable barrier 7. In the present embodiment, barrier 7 serves to prevent soil and liquid water from entering chamber 5 and comprises a grille 8 for mechanical stability and a membrane 9. Membrane 9 is permeable for vapour but not for water. Grille 8 can e.g. be formed by a plurality of bars or a perforated plate arranged at the bottom of housing 2.

Second opening 4 provides a passage from chamber 5 to ambient air. In the embodiment of Fig. 1 it is not covered by any barrier, but it may also be covered by a barrier (including a grille 8 and/or a membrane 9), in particular for mechanically protecting the interior of chamber 5, as long as the barrier is permeable for vapour.

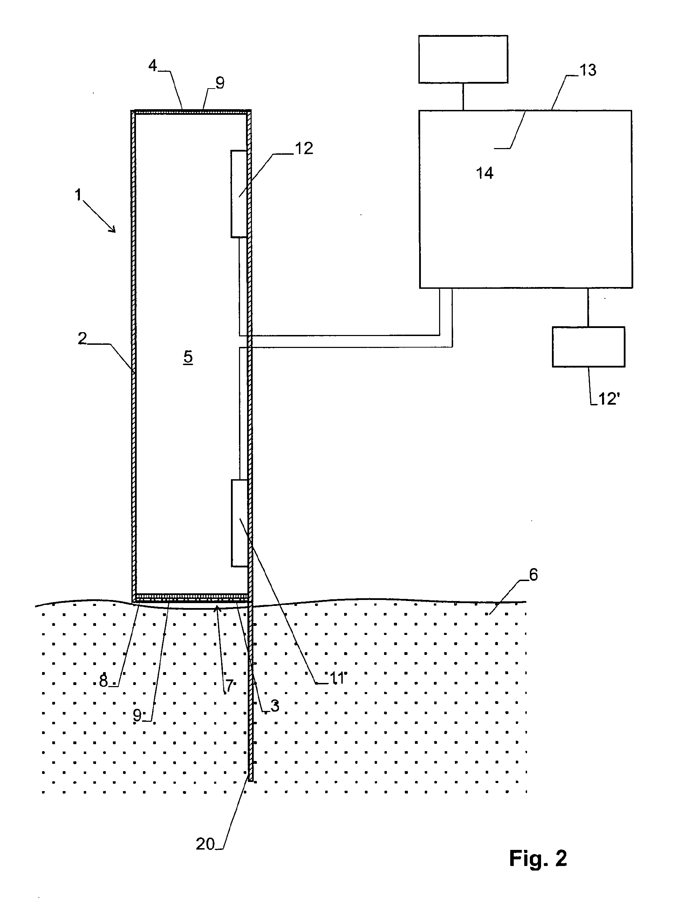

Closing second (upper) opening 4 by a membrane 9 (as it will be shown with reference to the second embodiment of Fig. 2) has been found to increase the measurement accuracy because it shield the humidity sensors from convective motion or drafts of the ambient air, thereby increasing signal accuracy. Also, it prevents water from entering chamber 5, e.g. during irrigation.

The membrane 9 used for covering the first and/or second opening is, as mentioned, advantageously made from a material that is permeable for vapour but not for (liquid) water. Advantageously, membrane 9 is of a hydrophobic material, such as polytetrafluoroethylene (PTFE) or PP (polypropylene), having pores in the micro-or nanometer scale.

In the embodiment of Fig. 1, two humidity sensors 11, 12 are arranged within chamber 5. They measure the humidity at two spaced-apart locations within chamber 5. The location monitored by first humidity sensor 11 is closer to first opening 3 than the location monitored by second humidity sensor 12.

The humidity sensors are designed to measure the level of humidity in air. They may e.g. be humidity sensors based on capacitive measurements such as the sensor disclosed in US 6 690 569. Each sensor further comprises a temperature sensor.

The humidity and temperature signals from each sensor 11, 12 are fed to a control unit 13, which may be mounted in probe 1 or located remotely from the same. In the latter case, the data transfer between probe 1 and control unit 13 may be carried out by means of a wireless or wire-bound communication.

The relative humidity measured by each sensor 11, 12 can be converted to an absolute humidity by means of the measured temperatures, thereby taking account of a temperature difference between the location of the two sensors. Control unit 13 can then compare the two humidities for deriving soil moisture content.

If the soil is dry and in an equilibrium state with the environment, the humidity at both sensors 11 and 12 is the same. If the soil is humid, there is a humidity gradient in the chamber 5 and a diffusion of humidity takes place. Hence, the humidity at first sensor 11 will be larger than the humidity at second sensor 12. The difference between the humidities is a measure for the soil moisture content in the soil. Further corrections can be applied to the measured difference, e.g. for compensating temperature-dependent effects.

If the device is used for irrigation control, control unit 13 can issue an alert for indicating an insufficient soil moisture content. The alert can e.g. be displayed on a display 14 and/or be used to initiate an automatic irrigation procedure.

In the embodiment of Fig. 1, both humidity sensors 11 and 12 are located in chamber 5. Alternatively the second humidity sensor 12 can also be located to measure the ambient humidity, which can then be used as a reference humidity to be compared to the humidity measured by first humidity sensor 11 in chamber 5.

For example, second humidity sensor 12 can be mounted to the outside of housing 1, or it may be placed at a location remote from probe 1, e.g. together with control unit 13, as indicated by reference numeral 12' in Fig. 1.

If control unit 13 with its own second humidity sensor 12' is located remote from probe 1, the same control unit 13 can monitor a plurality of probes 1, each of which comprising a single first humidity sensor only.

The second humidity sensor can be completely dispensed with if it is assumed that ambient humidity is at a fixed level. In that case, the humidity measured by first humidity sensor 11 alone is sufficient to assess the state of irrigation of the soil, e.g. by comparing the measured humidity to a fixed threshold value.

In the embodiment of Fig. 1, housing 2 is partially embedded in the soil. Alternatively, housing 2 can be placed outside the soil with its first opening 3 being in contact with the soil surface. Part of the excess moisture in the soil will still enter chamber 5 by diffusion and cause a humidity gradient therein.

In particular if housing 2 is not to be embedded into the soil but is intended to rest on the surface of the same, first opening 3 can be designed to be larger than second opening 4 in order to collect the moisture from a larger area of soil surface and in order to prevent the device from falling over easily.

Barrier 9 of the shown embodiment consists of two elements, namely grille 8 and membrane 9. The functions of these two elements can also be combined into one element, e.g. a sufficiently sturdy membrane. Furthermore, membrane 9 can be dispensed with, e.g. if it is assumed that humidity sensor 11 will, as a rule, not be below the soil's waterline. Similarly, grille 8 can be dispensed with, e.g. if it is assumed that soil entering chamber 5 will not interfere with the operation of first humidity sensor 11.

Fig. 2 shows an embodiment of the device that is suited for a placement above soil 6. In addition to the embodiment of Fig. 1, it comprises a fixation member 20, e.g. in the form of a stick, affixed to tubular housing 2 and extending beyond first opening 3. Fixation member 20 can be embedded in soil 6 for mechanically stabilizing the position of the device with the first opening 3 located at the top of the soil.

As can be seen, the second opening 4 of the device of Fig. 2 is closed by a membrane 9 for improved protection and measurement accuracy as mentioned above.

The positioning of the sensor or sensors within chamber 5 is fairly flexible. If two sensors are used, second sensor 12 should be on a downstream side (as seen along the diffusion gradient) of first sensor 11.

The sensors can be placed to communicate with chamber 5 through ducts, they can be flush with the walls of chamber 5, or they can project into chamber 5. However, the sensors should be placed such that they do not block the gas exchange between first opening 3 and second opening 4.

It has been found that the sensitivity of the device is increased if tubular housing 2 is of a material having low thermal conductivity, such as plastics. This is attributed to the fact that a housing with high thermal conductivity tends to equalize the temperatures at the locations of the two humidity sensors, thereby decreasing the humidity concentration gradient.

The soil moisture content can, as mentioned, be determined by comparing the two humidity values measured by the sensors. This comparison can e.g. involve the calculation of the difference between the humidities. The value calculated in this way can then to be compared to a threshold value to yield a Boolean signal indicating if an irrigation is required or not. However, the calculation may also involve any other linear or non-linear function of the two humidities or their difference and may be used to give a more descriptive signal, which can e.g. describe a percentage of humidity remaining in the soil or a time estimate until the soil should be irrigated a next time. Suitable functions can e.g. be derived by calibration measurements.

## Claims

1. A device for determining soil moisture content comprising a housing (2) and at least a first humidity sensor (11), said device being **characterized by**
a first opening (3) in said housing (2) to be brought onto or into the soil,
a second opening (4) in said housing (2) to be located outside said soil and
a chamber (5) in said housing (2) extending between said first opening (3) and said second opening (4), said chamber (5) forming a passage for allowing a diffusion of humidity between said first and said second openings (3, 4),
wherein said first humidity sensor (11) is located to measure the humidity at a first location in said chamber (5).

2. The device of claim 1 wherein said device is adapted to compare a humidity measured by said first humidity sensor (11) to a reference humidity.

3. The device of any of the preceding claims further comprising a second humidity sensor (12), wherein said device is adapted to compare the humidities measured by said first and said second humidity sensors (11, 12).

4. The device of claim 3 wherein said second humidity sensor (12) is located to measure the humidity at a second location in said chamber (5), which second location is at a larger distance from said first opening (3) than said first location.

5. The device of claim 3 wherein said second humidity sensor (12') is located to measure an ambient humidity.

6. The device of any of the preceding claims further comprising a temperature sensor, and in particular wherein each humidity sensor (11, 12) comprises a temperature sensor.

7. The device of any of the preceding claims further comprising a humidity permeable barrier (7) located at said first opening (3) for preventing an entry of soil into said chamber (5) through said first opening (3).

8. The device of any of the preceding claims further comprising a humidity permeable barrier (7) located at said second opening (4) for preventing an entry of soil or liquid water into said chamber (5) through said second opening (4).

9. The device of any of the claims 8 or 9 wherein said barrier (7) is permeable for vapour but impermeable for water

10. The device of claim 9 wherein said barrier (7) comprises a porous membrane (9) of a hydrophobic material.

11. The device of any of the preceding claims wherein said device is adapted to generate a signal indicative of an insufficient soil moisture content.

12. The device of any of the preceding claims wherein said housing (2) has tubular shape.

13. The device of any of the preceding claims further comprising a fixation member (20) mounted to said housing (2) and extending beyond said first opening (2) for fixing said device in soil with said first opening (2) located at a top of said soil.

14. Use of the device of any of the preceding claims for determining soil moisture content.

15. Use of claim 14 wherein said device is located on the soil with said first opening (2) located at a top of the soil.
